# EUROPEAN PATENT SPECIFICATION

(11) **EP 3 634 544 B1**
(45) Date of publication and mention of the grant of the patent: **05.05.2021**
(21) Application number: 18732411.6
(22) Date of filing: 25.05.2018
(51) Int. Cl.: A61M 5/24, A61M 5/32

(54) **INJECTOR DEVICES**
INJEKTORVORRICHTUNGEN
DISPOSITIFS D'INJECTION

(30) Priority: 07.06.2017 GB 201709072
(43) Date of publication of application: 15.04.2020
(73) Proprietor: NDM Technologies Ltd, Leicestershire LE11 3QF (GB)
(72) Inventor: CHOWDHURY, Dewan Fazlul Hoque, Loughborough Leicestershire LE11 1PN (GB)
(74) Representative: Serjeants LLP
(86) International application number: PCT/GB2018/051436
(87) International publication number: WO 2018/224805

(56) References cited:
- EP-A1- 1 949 927
- WO-A1-2016/196518
- WO-A1-2017/060394

## Description

### Technical field

The invention relates to disposable needle hubs for injector devices. Such hubs comprise at one end a needle used to administer drugs to a subject and at the other end a needle used to pierce the septum of a drug cartridge.

### Background

Injector devices used to administer drugs to humans or animals can be described as having two broad components in addition to the body of the injector device itself: a drug-filled disposable cartridge or reservoir and a disposable needle hub. The drug-filled cartridge usually has a rubber septum at the proximal end and a rubber plunger at the distal end. The needle hub generally has a thin needle at the proximal end, which pierces the skin of the patient, and a thicker, stronger needle at the distal end, which pierces the septum at the proximal end of the drug reservoir.

The same arrangement may be used for devices for withdrawing samples (e.g. blood samples) from a subject into a reservoir and the use of "injector", "drug cartridge" and similar terms in this specification is not intended to exclude such applications of the invention.

From a cost perspective it is beneficial to use a single needle for both the distal and proximal ends, consisting of the same diameter throughout. However, if the needle is of a high gauge, i.e., thin and often somewhat flexible, then it may buckle and bend when it is pressed into the septum of the drug reservoir due to the hardness of the septum, or due to rapid and high forces being applied when the cartridge and the needle hub are pushed together. The risk of buckling is particularly acute if the needle is intended to curve around an arc, such that the needle portion piercing the skin is not in line with an axis of the needle portion entering the drug cartridge. In this way the two ends of the needle may be mutually inclined at an angle, which is usually a right angle.

This arrangement is very useful where the device is intended to be applied flat on the skin rather than in the form of an elongated device applied vertically to the skin, with the associated psychological impact of injections and needle phobia. There exists a need for a device that allows controlled movement of the needle into the septum, or the septum over the needle, without high or rapid forces being applied, while maintaining the desired alignment of the needle to prevent buckling. WO 2017/060394, WO 2016/196518 and EP1949927 disclose injector devices. WO 2017/060394 discloses features falling under the preamble of claim 1.

### Summary of the invention

The invention provides an injector device as defined in claim 1.

The invention further provides a method of coupling a drug cartridge to an injector device as defined in claim 11.

Features of the invention that preferred but not essential are defined in the dependent claims.

Although the invention is described in terms of moving the neck of the drug cartridge into the collar of the injector device, it will be understood that only their relative movement is important so the injector device may equally be pushed onto the neck of the drug cartridge.

In this specification, "disposable" is used in relation to the needle and/or needle hub to indicate that the component will normally be replaced after each use; and in relation to the drug cartridge to indicate that the component will normally be replaced after each use or when it is empty. "Non-disposable" is used in relation to the housing of the injector device to indicate that the component will normally be retained for use on multiple occasions with different needle hubs and/or drug cartridges.

The device is suitable for use with straight or angled single-needle hubs where, without support, the needle would not have the integrity to remain firm when being inserted into a septum due to the hardness of the septum material relative to the hardness of the needle. The needle consists of a bore and an outer wall, whereby the outer wall and bore are continuous and constant throughout the length of the needle or may be slightly tapered to make one end thinner than the other. Preferably, though not an essential feature, the needle hub comprises a single length of needle of constant internal and external diameter as this is significantly cheaper to manufacture.

The needle may curve through a slight angle or an entire right angle; in such case the outer case of the needle hub may be used to secure the needle without the use of glue, using only sonic welding, laser welding or another form of fixing not based on chemical adhesives. Where the needle is completely straight, the needle is liable to be forced out of its casing longitudinally if an additional chemical adhesive is not used, however an angled needle will have significantly more friction to overcome due to the angled contacts between the needle and the inner case walls. The absence of glue reduces a process step and an additional material and hence the overall unit cost.

The invention further provides a needle aligning device which provides resistance and therefore controlled movement of the septum portion of a drug reservoir, prior to and after the insertion of the needle into and through the septum of the drug reservoir, intended to provide fluid communication between the contents of the reservoir and the needle. The needle aligning device consists of a support member that may be either directly attached to the needle hub or a separate component that is positioned adjacent to the septum-piercing portion of the needle in the needle hub, such that in both cases the rest position of the needle support member is close to the tip or extends beyond the tip of the needle, and gradually retracts as soon as the septum on the drug reservoir comes into contact with the tip of the needle and the needle inserts into the septum. Thereafter the needle support member will collapse to allow the required length of the needle to pierce through the septum to provide the fluid communication path with the drug reservoir. Collapsing of the needle support member will provide frictional force, through contact between the housing of the drug reservoir and the walls of the needle hub, or a resistant force as the support member collapses. It is preferable that the needle support member collapses in a manner, such as by folding against the inner wall, such that the length of the needle does not have to be elongated to accommodate the collapse, as longer needles lead to higher forces being required to inject the liquid through them.

### The drawings

Figures 1A and 1B are cross section schematics of a needle hub according to a first embodiment of the invention, showing a needle support member positioned in a rest position and an activated position respectively.
Figure 2 is an expanded view of the needle and needle support member of the first embodiment.
Figure 3A is a cross section schematic of a needle hub according to the first embodiment of the invention, with its needle touching the septum of a drug reservoir.
Figure 3B is the same view as Figure 3A, showing the drug reservoir having been pushed further into the needle hub.
Figure 4A is a cross section schematic of a needle hub and a drug reservoir aligned in an external housing of an injector device according to the invention.
Figure 4B is the same view as Figure 4A, showing the drug reservoir having been guided by the housing into engagement with the needle hub.
Figures 5A and 5B are cross section schematics of a needle hub according to a second embodiment of the invention, showing a needle support member positioned in a rest position and an activated position respectively.

### Description of preferred embodiments

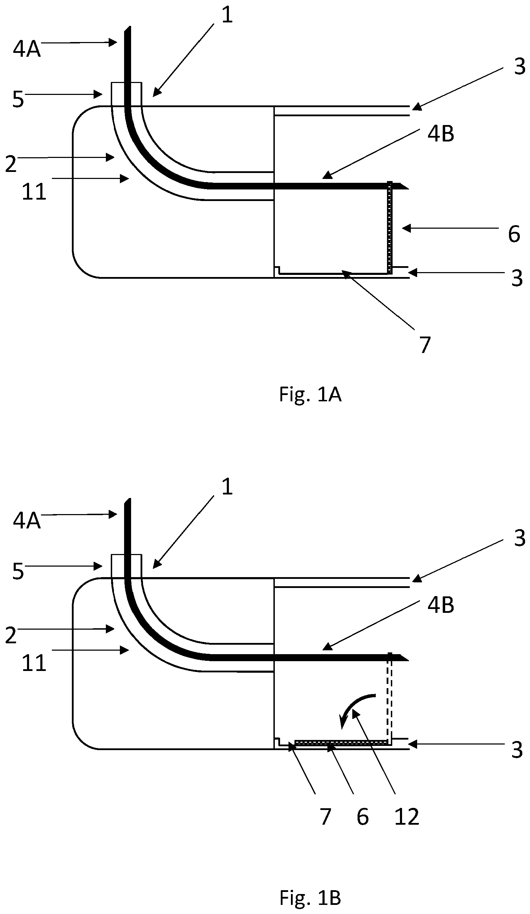

Figure 1A is a cross section schematic of a needle hub, which shows a needle 4 curved through an arc 11, within a housing 1. The housing 1 of the needle hub consists of two parts, or two halves, which enclose the needle 4. The proximal end of the needle 4A intended to pierce the skin is at a right angle to the distal end of the needle 4B. The distal end 4B is housed within a shroud or needle hub collar 3, a region within which the neck of a drug reservoir would fit and where the needle 4B pierces the septum of the drug reservoir to provide fluid communication between the contents of the drug reservoir and the needle 4 in the needle hub. The needle 4 does not have to be in the centre of the collar 3.

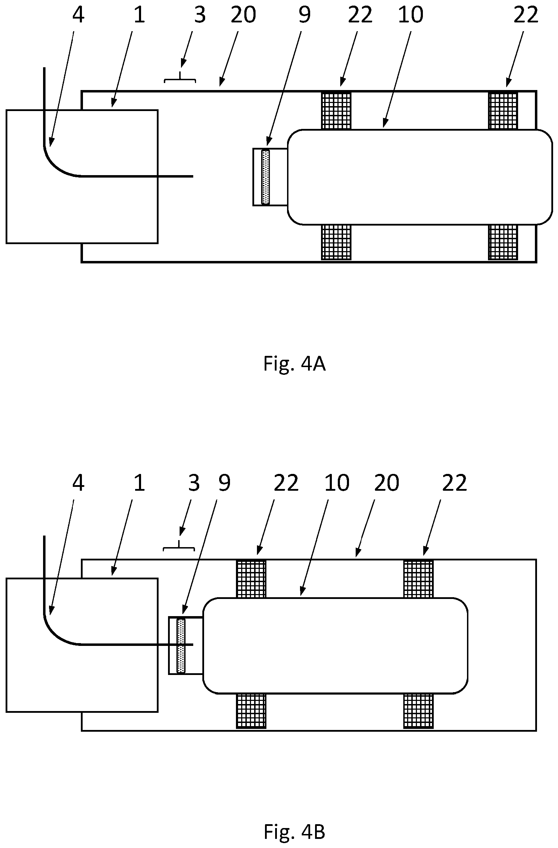

It will be understood that this collar 3 does not necessarily have to be part of the disposable needle hub and instead may be a feature of a permanent housing of the injector device (shown in Figure 4), within the region where the needle hub and drug reservoir interface. The collar also does not have to be a physical structure that surrounds the needle: any structure may be used to align the drug cartridge with the needle and to guide them towards one another and such a region where the needle and drug cartridge meet satisfies the definition of a collar/collar region for the present invention. The drug reservoir (which does not form part of the invention) may be a standard glass or polymeric vial, or a syringe or a compressible or collapsible reservoir, consisting of at least one septum which is to be pierced to enable fluid communication between the contents of the reservoir and a needle. The septum may be composed of natural or synthetic rubber, silicone or polymeric material.

A needle support member 6 is shown attached to the needle hub collar 3, allowing the distal end of the needle 4B to rest upon it. If the needle is curved through an angle, the support mechanism will be positioned to oppose the resilient force that tends to restore the needle to a straight configuration. This allows the needle 4B to rest within the support member 6 with some degree of downward force (in the illustrated orientation). In the case of a straight needle (or otherwise), two or more needle support members 6 could be distributed around the circumference of the needle hub to provide support for the needle 4 from different sides.

There is a recess 7 within the collar 3 of the needle hub intended to allow the support member 6 to be collapsed against the wall of the collar 3, to accommodate the entry of the neck of the drug reservoir into the needle hub collar 3. Arrow 12 in Figure 1B shows the movement of the support member 6 from its rest position (in dashed lines) to its activated position within the recess 7 (in solid lines). The support member 6 may be connected to the collar 3 by an articulated hinge or a living hinge to allow the member 6 to fold out of the way of the drug reservoir. The hinge may be designed to offer some resistance to the insertion of the reservoir, as described below. Additionally or alternatively, the displaced support member 6 may protrude slightly from the recess to offer frictional resistance as the reservoir pushes past it.

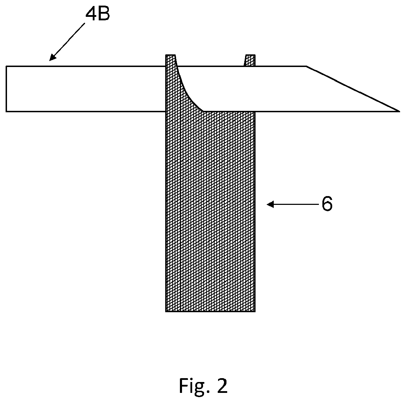

Figure 2 is a schematic illustration of the needle 4B resting on the needle support member 6, which has a concave tip to prevent the needle from slipping off. The support member 6 is positioned adjacent to the tip of the needle 4B such that it continues to support the needle until the tip of the needle 4B has pierced the septum of the drug reservoir and penetrated sufficiently that the support is now provided by the septum itself. At this point the support member function becomes one of purely providing a resistive force against which the drug reservoir neck (containing the septum) moves so as to prevent any rapid and sudden movement, thus reducing the chance of the needle buckling.

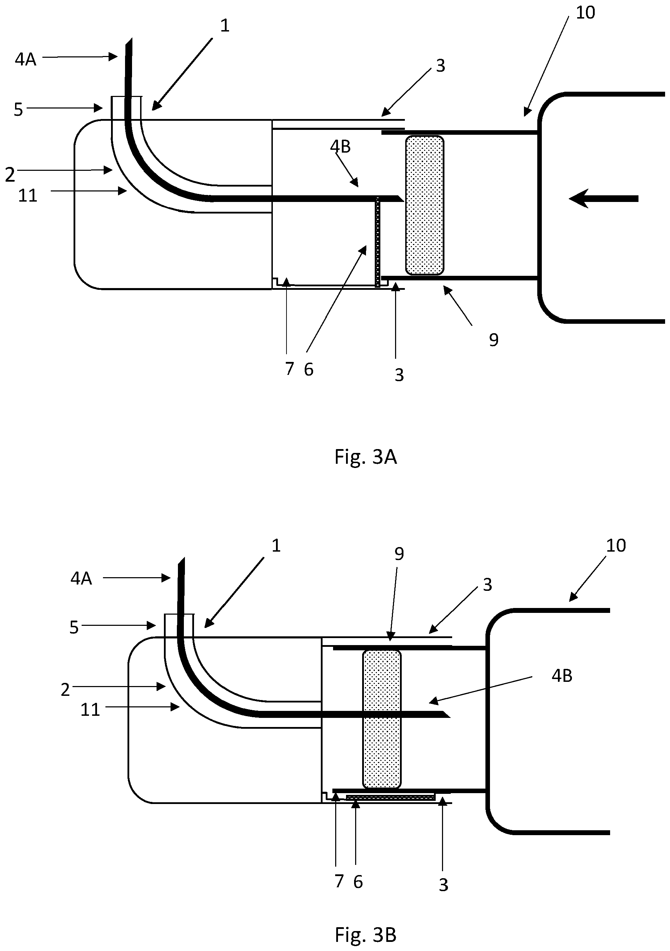

Figure 3A is a cross section schematic similar to Figure 1A, additionally showing the neck 10 of a drug reservoir fitted into the needle hub collar 3. A solid arrow indicates the direction of travel of the drug reservoir and in Figure 3A the septum 9 contained in the neck 10 of the drug reservoir is just in contact with the needle tip 4B.

Figure 3B depicts the same arrangement as Figure 3A at a later stage, with the neck 10 of the drug reservoir in its fully engaged position such that the needle 4B has pierced the septum 9 to the extent required. A pre-determined stop positon may be defined, for example by a shoulder (not shown) in the collar 3 of the needle hub. At this point the needle support member 6 is now fully collapsed in the recess 7 of the collar 3.

Figures 4A and 4B show how the disposable needle hub 1 and drug cartridge 10 may be supported and guided by a non-disposable external housing 20 of the injector device. The drug cartridge 10 is schematically shown as mounted on a carriage or bearings 22, on which the cartridge 10 can slide along the axis of the injector device until the needle 4 of the needle hub 1 pierces the septum 9 of the drug cartridge 10 within the collar region 3 of the device. The external housing 20 of the device can take many forms. Typically, the housing 20 encloses the needle hub 1 and the drug cartridge 10 except at their ends but it can be left at least partly open to allow manual access for sliding the drug cartridge along. Alternatively, the housing 20 may provide no more than a surface to rest against the skin of a patient and a track or other means for aligning and engaging the needle hub 1 and the cartridge 10. As seen in Figures 1 to 3, if guidance is provided by a collar 3 of the needle hub 1, the housing 20 can be omitted entirely.

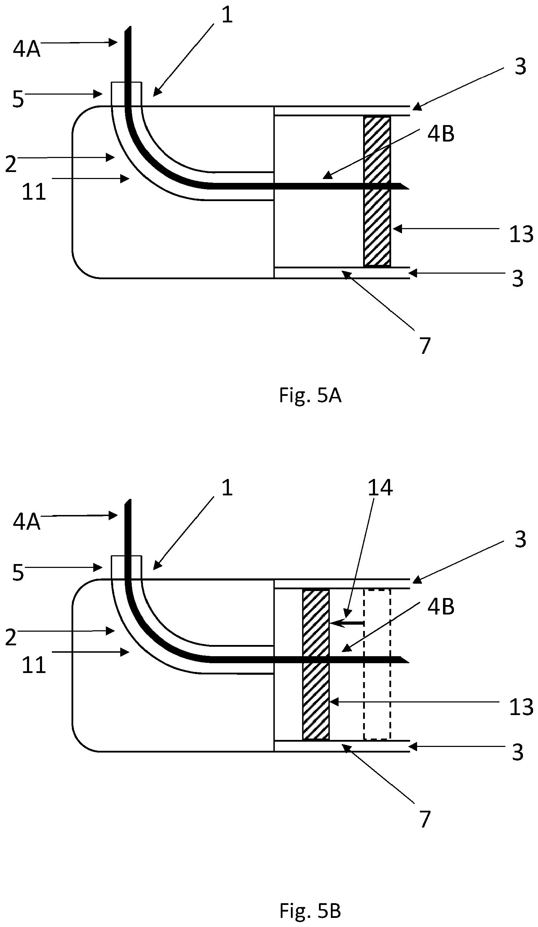

Figures 5A and 5B show a second embodiment of the invention, in which the needle support member is not attached to the needle hub collar 3 but is a standalone component such as a washer 13 with a central bore through which the distal end of the needle 4B protrudes. Arrow 14 in Figure 4B shows how, as the drug reservoir (not shown here) is inserted, it presses the washer 13 to slide into the collar 3 from a rest position (in dashed lines) to an activated position (in solid lines). The needle 4B must provide sufficient length to accommodate the washer 13 sliding along it and also to puncture the septum of the drug reservoir and provide fluid communication with the contents. It is a potential disadvantage of this embodiment that it may require an elongation of the needle 4B, as the longer a needle is, the greater the force required to inject a liquid through it for a given rate of injection/flow. This is not desired as it can lead to large forces that an injector device cannot cope with or lead to injury to humans when large forces are used to push a plunger or compress the contents of a drug reservoir to force the contents out, by way of potentially dislodging the needle 4A from the skin and leading to scraping of the skin.

The needle support member of any of the embodiments provides controlled resistance against the movement of the neck of the drug reservoir, to prevent the septum from being too rapidly pushed over the needle 4B or needle 4B pushed into the septum as this could lead to buckling and damage of the needle. This resistance can be attained in the case of the first embodiment by the needle support member 6 having a wall thickness and stiffness that requires a certain minimum force to cause it to collapse into the recess 7. In the second embodiment, the washer-like support member 13 or the inner walls of the collar 3 (or both) may be provided with surface features, or protrusions, that may be irregular or around the entire perimeter, to increase the sliding friction between them.

In a variant of the illustrated embodiments, the needle support member 6,13 in its rest position may cover the tip of the needle 4B, before the support member starts to be displaced by insertion of the drug cartridge 10. This helps to protect the needle tip and to avoid the risk of scratch injuries from the needle.

The illustrated embodiments show a needle that curves through a 90° arc between the proximal end 4A and the distal end 4B, whereby the drug cartridge 10 can lie flat against the skin of the subject as the proximal end of the needle 4A is inserted perpendicularly into the skin. It is important that the radius of the arc 11 should be sufficiently large to prevent the needle from becoming dented or leading to narrowing of the inner bore as it is formed into the arc. For example a fine, 33-gauge needle, having an inner diameter of 0.11mm and an outer diameter of 0.21mm, was observed to be physically deformed when bent around any arc radius of less than 3mm. A 30-gauge needle, having an inner diameter of 0.16mm and an outer diameter of 0.31mm produced no flow when bent around an arc radius of 5mm, which suggests that the inner bore was narrowed or occluded. From this point of view, a greater arc radius is to be preferred.

On the other hand, a greater arc radius may require a greater overall length of the needle, which increases the force needed to force liquid through, as shown in the following table based on experiments conducted with a 33-gauge needle.

| **Needle length (mm)** | **Average force required (N)** |
|---|---|
| 16 | 3.5 |
| 20 | 6.5 |
| 50 | 16.0 |

For a 33-gauge needle, an arc radius of 3mm or 4mm was not seen to obstruct the bore of the needle. This can be accommodated within an overall needle length of approximately 20mm, which permits flow through the needle with an acceptable level of force.

## Claims

1. An injector device (1) comprising:
a needle (4) having a proximal end (4A) for insertion into the skin of a patient and a distal end (4B) for insertion through a septum (9) in the neck of a drug cartridge (10), the alignment of the distal end (4B) defining an axis;
a collar region (3) for receiving the neck of the drug cartridge (10); and
a needle support member (6,13), **characterised in that** the needle support member (6, 13) supports the needle (4) at a rest position close to the distal end (4B) before the neck of the drug cartridge (10) is received in the collar region (3), and which is displaceable by the neck of the drug cartridge (10) relative to the needle (4), such that the needle support member (6,13) moves away from the rest position as the neck moves into the collar region (3).

2. An injector device (1) according to claim 1, wherein the needle support member (6) is attached to the collar (3) via a hinge.

3. An injector device (1) according to claim 2, wherein the collar (3) comprises a recess (7) for receiving the needle support member (6) when it has been displaced by the drug cartridge (10).

4. An injector device (1) according to claim 2 or claim 3, wherein the needle (4) curves such that the proximal end (4A) is aligned at an angle relative to the axis, and wherein the needle support member (6) is attached to the collar (3) on the opposite side of the axis from the proximal end (4A).

5. An injector device (1) according to any preceding claim, comprising two or more needle support members (6) distributed around the distal end (4B) of the needle (4).

6. An injector device (1) according to claim 1, wherein the needle support member (13) surrounds the distal end (4B) of the needle (4) and wherein the needle support member (13) is capable of sliding in the collar (3), parallel to the axis, as the needle support member (13) is displaced by the drug cartridge (10).

7. An injector device (1) according to claim 6, further comprising protrusions for increasing friction between the needle support member (13) and the collar (3).

8. An injector device (1) according to any preceding claim, wherein the proximal end (4A) and the distal end (4B) are opposite ends of a single needle (4) of constant diameter.

9. An injector device (1) according to claim 8, wherein the needle (4) curves through an arc such that the proximal end (4A) is aligned substantially at a right angle to the axis.

10. An injector device (1) according to any preceding claim, wherein the needle (4) is removably mounted in the device.

11. A method of coupling a drug cartridge to an injector device (1) of any of the previous claims comprising the steps of:
using a needle support member (6,13) to support a needle (4) within a collar (3) of the injector device (1) at a rest position close to a distal end (4B) of the needle (4);
inserting a neck of the drug cartridge (10) into the collar (3);
piercing a septum (9) of the drug cartridge (10) with the distal end of the needle (4); and
pushing the drug cartridge further into the collar (3) to displace the needle support member (6,13) relative to the needle (4), such that the needle support member (6,13) moves away from the rest position.

12. A method according to claim 11, wherein the step of displacing the needle support member (6) comprises bending the needle support member (6) about a hinge.

13. A method according to claim 11, wherein the step of displacing the needle support member (13) comprises sliding the needle support member (13) parallel to an axis of the collar (3).

## Patentansprüche

1. Injektorvorrichtung (1), umfassend:
eine Nadel (4) mit einem proximalen Ende (4A) zum Einführen in die Haut eines Patienten und einem distalen Ende (4B) zum Einführen durch ein Septum (9) in den Hals einer Arzneimittelpatrone (10), wobei die Ausrichtung des distalen Endes (4B) eine Achse definiert;
einen Kragenbereich (3) zur Aufnahme des Halses der Arzneimittelpatrone (10); und
ein Nadelträgerelement (6, 13), **dadurch gekennzeichnet, dass** das Nadelträgerelement (6, 13) die Nadel (4) in einer Ruheposition nahe dem distalen Ende (4B) abstützt, bevor der Hals der Arzneimittelpatrone (10) in dem Kragenbereich (3) aufgenommen wird, und das durch den Hals der Arzneimittelpatrone (10) relativ zu der Nadel (4) verschiebbar ist, so dass sich das Nadelträgerelement (6, 13) von der Ruheposition wegbewegt, wenn sich der Hals in den Kragenbereich (3) bewegt.

2. Injektorvorrichtung (1) nach Anspruch 1, wobei das Nadelträgerelement (6) über ein Scharnier an dem Kragen (3) befestigt ist.

3. Injektorvorrichtung (1) nach Anspruch 2, wobei der Kragen (3) eine Ausnehmung (7) zur Aufnahme des Nadelträgerelements (6) aufweist, wenn dieses durch die Arzneimittelpatrone (10) verschoben wurde.

4. Injektorvorrichtung (1) nach Anspruch 2 oder Anspruch 3, wobei die Nadel (4) so gekrümmt ist, dass das proximale Ende (4A) in einem Winkel relativ zur Achse ausgerichtet ist, und wobei das Nadelträgerelement (6) am Kragen (3) auf der dem proximalen Ende (4A) gegenüberliegenden Seite der Achse befestigt ist.

5. Injektorvorrichtung (1) nach einem vorstehenden Anspruch, umfassend zwei oder mehr Nadelträgerelemente (6), die um das distale Ende (4B) der Nadel (4) verteilt sind.

6. Injektorvorrichtung (1) nach Anspruch 1, wobei das Nadelträgerelement (13) das distale Ende (4B) der Nadel (4) umgibt und wobei das Nadelträgerelement (13) in der Lage ist, in dem Kragen (3) parallel zur Achse zu gleiten, wenn das Nadelträgerelement (13) durch die Arzneimittelpatrone (10) verschoben wird.

7. Injektorvorrichtung (1) nach Anspruch 6, die weiter Vorsprünge zur Erhöhung der Reibung zwischen dem Nadelträgerelement (13) und dem Kragen (3) umfasst.

8. Injektorvorrichtung (1) nach einem vorstehenden Anspruch, wobei das proximale Ende (4A) und das distale Ende (4B) gegenüberliegende Enden einer einzelnen Nadel (4) mit konstantem Durchmesser sind.

9. Injektorvorrichtung (1) nach Anspruch 8, wobei die Nadel (4) durch einen Bogen gekrümmt ist, so dass das proximale Ende (4A) im Wesentlichen in einem rechten Winkel zur Achse ausgerichtet ist.

10. Injektorvorrichtung (1) nach einem vorstehenden Anspruch, wobei die Nadel (4) in der Vorrichtung abnehmbar angebracht ist.

11. Verfahren zum Ankoppeln einer Arzneimittelpatrone an eine Injektorvorrichtung (1) nach einem der vorstehenden Ansprüche, umfassend die Schritte:
Verwenden eines Nadelträgerelements (6, 13), um eine Nadel (4) innerhalb eines Kragens (3) der Injektorvorrichtung (1) in einer Ruheposition nahe einem distalen Ende (4B) der Nadel (4) zu halten;
Einführen eines Halses der Arzneimittelpatrone (10) in den Kragen (3);
Durchstechen eines Septums (9) der Arzneimittelpatrone (10) mit dem distalen Ende der Nadel (4); und
Drücken der Arzneimittelpatrone weiter in den Kragen (3), um das Nadelträgerelement (6, 13) relativ zur Nadel (4) zu verschieben, so dass sich das Nadelträgerelement (6, 13) von der Ruheposition weg bewegt.

12. Verfahren nach Anspruch 11, wobei der Schritt des Verschiebens des Nadelträgerelements (6) das Biegen des Nadelträgerelements (6) um ein Gelenk umfasst.

13. Verfahren nach Anspruch 11, wobei der Schritt des Verschiebens des Nadelträgerelements (13) das Gleiten des Nadelträgerelements (13) parallel zu einer Achse des Kragens (3) umfasst.

## Revendications

1. Dispositif d'injection (1) comprenant :
une aiguille (4) présentant une extrémité proximale (4A) pour une insertion dans la peau d'un patient et une extrémité distale (4B) pour une insertion à travers un septum (9) dans le col d'une cartouche de médicament (10), l'alignement de l'extrémité distale (4B) définissant un axe ;
une zone de collier (3) pour recevoir le col de la cartouche de médicament (10) ; et
un élément de support d'aiguille (6, 13), **caractérisé en ce que** l'élément de support d'aiguille (6, 13) supporte l'aiguille (4) dans une position de repos proche de l'extrémité distale (4B) avant que le col de la cartouche de médicament (10) soit reçu dans la zone de collier (3), et qui est déplaçable par le col de la cartouche de médicament (10) par rapport à l'aiguille (4), de telle sorte que l'élément de support d'aiguille (6, 13) s'éloigne de la position de repos tandis que le col se déplace dans la zone de collier (3).

2. Dispositif d'injection (1) selon la revendication 1, dans lequel l'élément de support d'aiguille (6) est fixé au collier (3) via une charnière.

3. Dispositif d'injection (1) selon la revendication 2, dans lequel le collier (3) comprend un évidement (7) pour recevoir l'élément de support d'aiguille (6) lorsqu'il a été déplacé par la cartouche de médicament (10).

4. Dispositif d'injection (1) selon la revendication 2 ou la revendication 3, dans lequel l'aiguille (4) se recourbe de telle sorte que l'extrémité proximale (4A) est alignée selon un angle par rapport à l'axe, et dans lequel l'élément de support d'aiguille (6) est fixé au collier (3) sur le côté opposé de l'axe à partir de l'extrémité proximale (4A).

5. Dispositif d'injection (1) selon une quelconque revendication précédente, comprenant deux éléments de support d'aiguille (6) ou plus répartis autour de l'extrémité distale (4B) de l'aiguille (4).

6. Dispositif d'injection (1) selon la revendication 1, dans lequel l'élément de support d'aiguille (13) entoure l'extrémité distale (4B) de l'aiguille (4) et dans lequel l'élément de support d'aiguille (13) est capable de coulisser dans le collier (3), parallèlement à l'axe, tandis que l'élément de support d'aiguille (13) est déplacé par la cartouche de médicament (10).

7. Dispositif d'injection (1) selon la revendication 6, comprenant en outre des saillies pour augmenter un frottement entre l'élément de support d'aiguille (13) et le collier (3).

8. Dispositif d'injection (1) selon une quelconque revendication précédente, dans lequel l'extrémité proximale (4A) et l'extrémité distale (4B) sont des extrémités opposées d'une aiguille unique (4) de diamètre constant.

9. Dispositif d'injection (1) selon la revendication 8, dans lequel l'aiguille (4) se recourbe par le biais d'un arc de telle sorte que l'extrémité proximale (4A) est alignée sensiblement selon un angle droit avec l'axe.

10. Dispositif d'injection (1) selon une quelconque revendication précédente, dans lequel l'aiguille (4) est montée de manière amovible dans le dispositif.

11. Procédé de couplage d'une cartouche de médicament à un dispositif d'injection (1) selon l'une quelconque des revendications précédentes comprenant les étapes consistant à :
utiliser un élément de support d'aiguille (6, 13) pour supporter une aiguille (4) au sein d'un collier (3) du dispositif d'injection (1) dans une position de repos proche d'une extrémité distale (4B) de l'aiguille (4) ;
insérer un col de la cartouche de médicament (10) dans le collier (3) ;
percer un septum (9) de la cartouche de médicament (10) avec l'extrémité distale de l'aiguille (4) ; et
pousser la cartouche de médicament plus profondément dans le collier (3) pour déplacer l'élément de support d'aiguille (6, 13) par rapport à l'aiguille (4), de telle sorte que l'élément de support d'aiguille (6, 13) s'éloigne de la position de repos.

12. Procédé selon la revendication 11, dans lequel l'étape de déplacement de l'élément de support d'aiguille (6) comprend la courbure de l'élément de support d'aiguille (6) autour d'une charnière.

13. Procédé selon la revendication 11, dans lequel l'étape de déplacement de l'élément de support d'aiguille (13) comprend le coulissement de l'élément de support d'aiguille (13) parallèlement à un axe du collier (3).
